# EUROPEAN PATENT SPECIFICATION

(11) **EP 2 801 375 B1**
(45) Date of publication and mention of the grant of the patent: **07.04.2021**
(21) Application number: 14167437.4
(22) Date of filing: 07.05.2014
(51) Int. Cl.: A61L 2/232, A61L 15/28, C07H 21/00, C08B 37/08, A61L 2/22, A61L 2/10

(54) **Device for sanitizing objects**
Vorrichtung zur Desinfektion von Gegenständen
Appareil pour la désinfection des objets

(30) Priority: 07.05.2013 IT MI20130737
(43) Date of publication of application: 12.11.2014
(73) Proprietor: SOL S.p.A., 20900 Monza (MB) (IT)
(72) Inventor: VALTOLINA, Daniele, I-23896 Sirtori (LC) (IT)
(74) Representative: Gislon, Gabriele

(56) References cited:
- EP-A2- 1 975 528
- WO-A2-2010/127296
- GB-A- 640 808
- US-A1- 2004 120 852
- DATABASE WPI Week 200312 Thomson Scientific, London, GB; AN 2003-126724 XP002729889, & KR 2002 0068794 A (CHOI Y S) 28 August 2002 (2002-08-28) & KR 2002 0068794 A (CHOI YOUNG SIN [KR]) 28 August 2002 (2002-08-28)

## Description

The present invention relates to a device for sanitizing objects, particularly medical devices in a hospital environment.

In a hospital or in a laboratory, a large variety of objects have to be brought to sterile conditions, i.e. to be decontaminated; some of them can be sterilized by methods involving an increase in temperature, whereas others, for example medical devices such as equipment comprising electrical or electronic parts, or devices made of a material which is not able to withstand the sterilization temperature, are subjected to a sanitization procedure. This procedure involves placing the objects to be decontaminated in a special container where the object is subjected to irradiation with UV lamps and it is contacted with biocidal liquids or gases.

Devices for the so-called sanitization of objects have been known for a long time. U.S. 3436173 describes a portable sanitizing device comprising a seal chamber for accommodating objects to be sanitized; the biocidal action is carried out by ethylene oxide which is supplied to the chamber and subsequently expelled through a filter at the end of the cycle.

EP 2136848 relates to a movable device for sanitizing probes or other devices which are attached to the end of a cable. WO 2004/034766 describes a portable decontamination unit, especially for letters and parcels, in which the biocidal action is carried out by UV lamps and hot air which is supplied to the decontamination chamber, possibly in combination with ozone or biocidal products.

KR 20020068794 describes a device for sanitizing banknotes in which a disinfectant based on plant extracts is used which is sprayed by means of a gas fed into a chamber placed under a reduced pressure by means of a vacuum pump. The vacuum is used to deliver the biocide into the chamber and to make it penetrate the bundles of banknotes. The above-described devices as well as similar ones known in the art have the problem that, if portable, they are not stand-alone and, in certain cases, they are too small in size to be effective for use in a hospital setting. Furthermore, the devices which make use of a vacuum, as in the case of the above-mentioned KR'794, require expensive facilities to be able to withstand and maintain the vacuum during the sanitization or decontamination procedure.

US 2004/0120852 discloses a disinfecting device for medical instruments, in particular endoscopes. In operation, the medical instruments are placed under pressure in a chamber and covered in a supercritical fluid. A vacuum source, causes a quick expansion of the fluid in the chamber. As an alternative, the chamber is opened to the external environment, so as to put the fluid at ambient pressure to cause the above mentioned expansion. The expansion causes an "explosion" of virus and bacteria.

EP 1975528 discloses a device for sanitizing ice machines. A disinfectant is mixed with pressurized air and supplied directly within the device by means of a nozzle.

WO2010/127296 discloses different embodiments of decontamination apparatuses. The decontamination apparatus of WO'296 is provided with an enclosure having at least two different kinds of decontamination system, among which there is a fogger for biocidal fluid. The apparatus is further provided with a system for evacuating or exchanging the atmosphere within the enclosure.

GB 640808 discloses a nebulization device for antiseptic or disinfecting fluids that may be used in hospitals.

The aim of the present invention is to solve the above mentioned problems and to provide a sanitizing device which doesn't require the use of vacuum and which ensures a reliable, effective sanitation in order to obtain sterile objects ready to be packaged into sterile containers at the opening of the device. Preferably, the device of the invention will be portable and able to perform all the functions of a fixed device without having to be connected to an electrical network. The portable device can be moved from a hospital department to another according to treatment needs of the products to be decontaminated.

This object is achieved by the present invention, which relates to a device for sanitizing objects which comprises a decontamination chamber, a source of biocidal fluid, means for feeding said fluid to said chamber and at least one source of compressed air, characterized according to claim 1.

The term "medical air" and/or "reconstituted air" is intended to define breathing air having the features (percentage of oxygen, nitrogen, moisture, ..) provided by the Official Pharmacopoeia, such as the Italian Official Pharmacopoeia, 10th Edition 1999 (General Chapter, pp. 3 - 5), and whose production is described, for example, in "Produzione di aria medicale presso le strutture ospedaliere" - Assogastecnici, 1st Edition, 2001, (http://www.sol.it/AreaClienti/solconsulting/consulting/A.1.pdf).

The air contained in the cylinder is used at the end of the decontamination process, when the atmosphere existing in the decontamination chamber and containing the biocidal product, is removed from the chamber itself and replaced with air fed from the cylinder. In this way, it is possible to avoid bringing air from the environment surrounding the sanitizing device or cabinet into the same; in fact, especially when operating in a hospital department or in a laboratory, the air existing in the environment may be polluted, e.g. by micro-organisms and, if fed into the decontamination chamber, it may re-contaminate the objects which have been just decontaminated. Using medical air or reconstituted air, i.e. breathing air with features as provided by the Official Pharmacopoeia, for the so-called purging of the chamber, will allow the decontaminated objects to be wrapped or packaged without substantially contacting the ambient air, i.e. contacting it for a very short time.

According to a preferred aspect of the invention, the biocidal fluid is a liquid, and the means for feeding it to the decontamination chamber comprise a nebulizer. In an exemplary embodiment, the nebulizer is fed with air from the interior of the decontamination chamber. Preferably, the device according to the invention further comprises means for expelling air from the decontamination chamber, said means comprising activated carbon and/or HEPA filters. In order to expel the contaminated air, fans are used which act from the inside and/or from the outside the chamber. According to a particular aspect of the present invention, both the step of expelling the contaminated air (purging) and the step of delivering the biocidal liquid, occur at ambient pressure. The biocidal liquid is nebulized and then circulated inside the decontamination chamber by means of at least one fan acting within the decontamination chamber during the nebulization of the biocidal liquid. In a preferred embodiment of the present invention, said at least one fan used for the step of recirculating the biocidal fluid within the chamber is also used for expelling air from the decontamination chamber; in this embodiment, when multiple fans are present, at least one of them is used to promote the expulsion of air through the filter(s).

The nebulizer device for the biocidal liquid is of a type known in the art. Suitable nebulizer devices are those based on ionization and those using a fan for supplying ambient air to a Venturi tube to which the biocidal liquid is fed in order to be nebulized. An example of a suitable nebulizer is marketed by Oxypharm® (Italy) under the trade name Nocospray.

In order to be completely stand-alone, the device is provided with a power source which is independent from the electric network, particularly, it is electrically fed by a hydrogen Fuel Cell unit provided with associated gas storage means (not shown) (such as metal hydrides and/or chemical hydrides and/or carbon nanostructures and/or pressure cylinders, ..) and/or a UPS and/or a storage battery, to be used when electrical power is not available and/or when the device is not carrying out sanitization cycles, for the operation of its electrical parts with an electrical power supply in situ; preferably, said UPS and/or said storage battery can be recharged by the Fuel Cell itself.

The device according to one of the previous embodiments may also comprise UV-C lamps (Ultraviolet Germicidal Emitters).

The device may further comprise an air compressor in addition to the aforementioned cylinder of medical air and/or reconstituted air.

Preferably, the device further comprises a control unit and means for programming the control unit which performs the sanitization or decontamination cycle. Preferably, the means for programming the control unit are selected from a display and a computer connectable with said control unit, or a combination thereof; the computer can replace the control unit.

The present invention encompasses all or part of the combinations of features and embodiments discussed above.

The device according to the present invention has numerous advantages over the prior art. As mentioned, the device can be made movable and stand-alone in a simple manner; this means that it can be moved from one place to another, for example from one department to another within a hospital, instead of moving the objects to be decontaminated, with the result of obtaining clear advantages. Furthermore, once the sanitization/decontamination procedures in a hospital or laboratory are finished concluded, the device can be moved to another hospital or laboratory, where it can perform decontamination procedures as required.

Therefore, the invention also provides a method for decontaminating objects in a healthcare environment such as, for example, a hospital, clinic or laboratory, which comprises using a device as above described and which is characterized according to claim 11. In a preferred embodiment of the process, the same device is used in different healthcare environments, and a step is provided in which the device is transferred from one to another of said environments.

A further advantage is given by the presence of a cylinder of medical air and/or reconstituted air as a source of compressed air; this feature leads to the ability to use sterile, dry air for purging the decontamination chamber by expelling air from the chamber through the filters and replacing it with medical air and/or reconstituted air. Moreover, the presence of the cylinder allows for power savings, as the air is already compressed: the filling of the decontamination chamber with the nebulized biocide doesn't require the presence of a compressor, and the decontamination process doesn't require the presence of a vacuum pump because it occurs at ambient pressure, as already mentioned.

When the decontamination process is carried out at ambient pressure, particularly with recirculation, certain advantages can be obtained such as, for example, the ability to safely decontaminate medical instruments which may contain, in their inside, sensitive parts (sensors) which could be damaged due to pressure changes. A further advantage is to simplify the design of the sanitizing device, for example by removing the security inputs/ outputs and valves thereof which are present in the known devices where the decontamination chamber is brought to a pressure which is lower or higher than ambient pressure.

These and other advantages will be apparent from the following description with reference to the drawings, which are attached by way of example and not of illustration, in which:
- Fig. 1 is a schematic, perspective view of an embodiment according to the invention;
- Fig. 2 is a schematic, perspective view of a second embodiment according to the invention;
- Figs. 3A and 3B are schematic, sectional views of a particular embodiment of the means for recirculating and expelling air during the step of nebulizing the biocidal liquid (Fig. 3A) and the step of purging the decontamination chamber (Fig. 3B), respectively.

With reference to these Figures, a sanitizing cabinet for decontamination of objects, such as medical instruments, according to a preferred aspect of the present invention, is referred to by reference numeral 1. The device is of a movable type and, to this purpose, the device comprises swivel wheels 14.

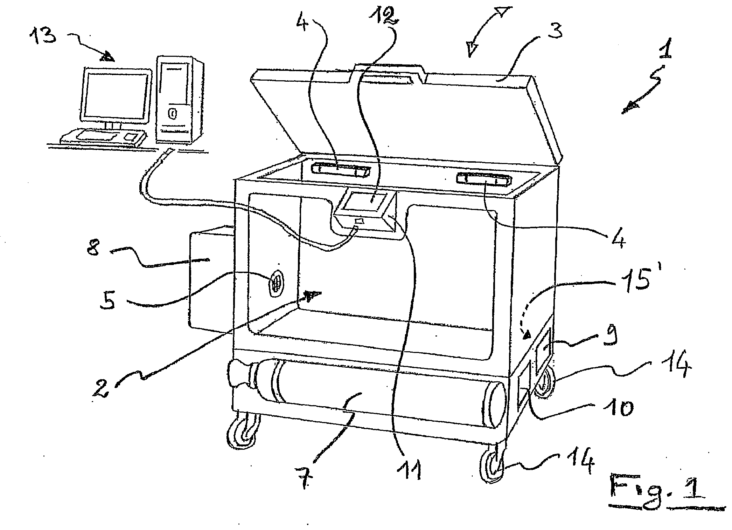

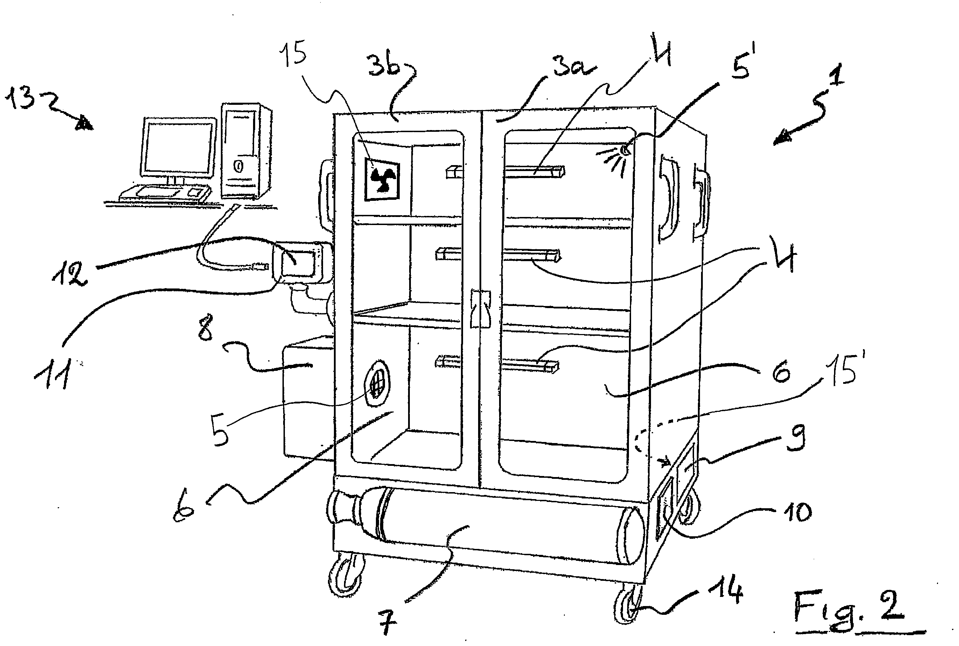

With reference to Figure 1, the sanitizing cabinet 1 for decontamination comprises a sanitizing chamber 2 in which at least one object or medical instrument to be decontaminated can be accommodated, at least one hermetic door 3, at least one germicidal lamp 4, preferably of a UV-C type, and at least one nozzle 5 for delivering the biocidal fluid to the chamber 2. In the shown embodiments, the biocidal fluid is a liquid and it is stored in a reservoir (not shown) which is housed in one or more portions 8 of the device along with one or more nebulizers. In the embodiment of Figure 2, a further nebulizer is shown, of which a nozzle 5' can be seen.

At least part of the body of the device is equipped with a transparent observation window 6: in the embodiment of Fig. 1, the window is formed in one of the walls of the device, and in the embodiment of Fig. 2, each of the two doors 3a and 3b is provided with a window 6.

With reference to Figure 2, the device 1 is provided with two nozzles 5, 5' for delivering the biocidal liquid by means of nebulizers. The nozzle 5' can be connected to the same nebulizer housed in the portion 8 of the device or, alternatively, it can be connected to a further nebulizer, such as an ionization-based nebulizer of the type discussed above. The device 1 is also provided with recirculating means comprising at least one fan 15 by which, during the decontaminating step, the air is recirculated within the decontamination chamber along with the biocidal fluid.

The device is also provided with a source of medical compressed air and/or reconstituted air, which is preferably a cylinder of compressed air. In the attached Figures, the cylinder 7 is shown external to the body of the device for increased convenience of reading, but the cylinder may be arranged either externally or internally thereto. As explained above, the compressed air in the cylinder 7 is used for the step of purging the chamber 2 at the end of the decontaminating step.

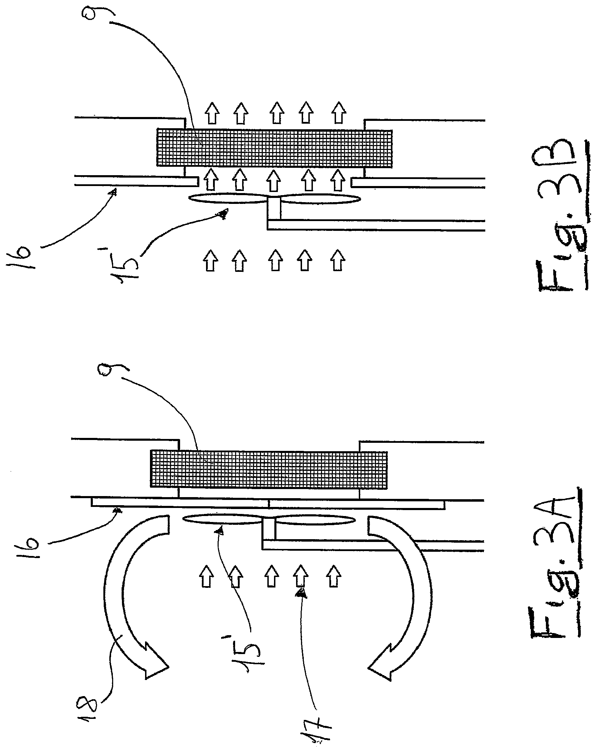

In this step, the mixture of air and biocidal fluid existing in the chamber 2 is expelled by expelling means comprising at least one fan 15' and filters 9. The contaminated air is then pushed outwardly from the device through the filters 9, which are of the type suitable to capture chemical pollutants and/or micro-organisms; suitable filters are selected from activated carbon filters and HEPA filters, and combinations thereof. In the embodiment shown in Figures, the expulsion of contaminated air is carried out by fans 15' (as shown in Figure 3B and known in the art) acting from the inside of the chamber. However, further embodiments are provided in which the expulsion of contaminated air is carried out by means of fans which act from the inside and/or from the outside of the chamber and which are dedicated to the expulsion.

Figures 3A and 3B show a particular embodiment of the air-expelling means in which at least one shutter 16 is provided as interposed between the fan 15' and the filter 9. The shutter 16 comprises one or more portions which are movable between an open position (as shown in Fig. 3B) and a closed position (as shown in Fig. 3A). Particularly, in the open position, the air flow generated by the rotation of the fan 15' is expelled from the contamination chamber towards the external environment through the filter 9. In the closed position, the shutter intercepts the air flow generated by the rotation of the fan 15'. The air flow (as indicated by the arrows 17 in Figure 3A) is reflected back by the shutter 16, thereby generating an air flow 18 which can be used as a recirculation flow during the sanitizing step. Preferably, the recirculating fans are activated at least during the nebulizing step; a further recirculating step is provided which can be used after the nebulizing step.

In other words, in this embodiment, the fan 15' can act both as an air-recirculation means during the nebulization of the biocidal liquid (while maintaining the shutter 16 in the closed position) and as an expelling means during the step of rinsing the decontamination chamber (while maintaining the shutter 16 in the open position).

In order to be completely stand-alone, the device is provided with a power source 10 which is independent from the electric network, particularly a hydrogen Fuel Cell unit provided with associated means for the storage of gases (such as metal hydrides and/or chemical hydrides and/or carbon nanostructures and/or pressure cylinders, ..) and/or a UPS and/or a storage battery, to be used when electrical power is not available and/or when the device is not carrying out sanitization cycles, for the operation of its electrical parts with an electrical power supply in situ; preferably, said UPS and/or said storage battery can be recharged by the Fuel Cell itself.

The device may further comprise an air compressor in addition to the aforementioned cylinder of medical air and/or reconstituted air.

As can be seen from the Figures, in the embodiments as shown, the device also comprises a control unit 11 provided with a graphic display 12 (possibly a GUI) and means for programming the control unit which performs the sanitization - i.e. decontamination - cycle. Preferably, the means for programming the control unit are selected from the display itself and a computer 13 connectable with said control unit, or a combination thereof; the computer can replace the control unit.

The "cabinet"-type embodiment of Fig. 2 comprises the same elements as set forth in fig.1, which are referred to by the same reference numerals. The embodiment of Fig. 2 has two doors and a plurality of shelves for supporting a plurality of objects to be decontaminated. A UV-C lamp for each compartment can be provided. The compartments are in fluid communication with each other in order to allow air and biocide to be recirculated as discussed above.

The process for performing the decontamination of objects in a healthcare environment provides for carrying out the loading and the decontamination in a known way with the use of a device as described above, and it also provides for a step of purging the chamber 2 at the end of the decontaminating step in which medical air and/or reconstituted air is delivered into said chamber 2. In a preferred embodiment of the process, the same device 1 is used in different healthcare environments, and a step is provided in which the device is transferred between said environments, either within a hospital or between different hospitals.

## Claims

1. A device (1) for sanitizing objects, comprising a decontamination chamber (2); a source (8) of biocidal fluid; means (5) for feeding said fluid to said chamber (2) and at least one source (7) of compressed air, **characterized in that** said compressed air is medical air, preferably said source being a cylinder (7) of medical compressed air.

2. A device (1) according to claim 1, wherein the biocidal fluid is a liquid and said means (5) for feeding it to said decontamination chamber (2) comprise at least one nebulizer.

3. A device according to any previous claim, further comprising means (15, 15') for recirculating air inside said chamber in order to diffuse said nebulized or gaseous biocide inside said chamber.

4. A device (1) according to any previous claim, further comprising means for expelling air from the decontamination chamber (2), said expelling means comprising activated carbon and/or HEPA filters (9).

5. A device according to claims 3 and 4, wherein said recirculating means comprise at least one fan (15, 15'), and wherein said fan or one of said fans (15') is positioned either adjacent to or in proximity of an activated carbon filter or a HEPA filter and it forms one of said means (9) for expelling air and biocide from said chamber (2).

6. A device according to claim 5, wherein a shutter member (16) for intercepting an air flow is arranged between said fan (15') and said filter (9), said shutter member being movable in order to open or close the air passage through said filter (9) to the outside.

7. A device (1) according to any previous claim, further comprising a power source (10) which is independent from the electric network, preferably a hydrogen Fuel Cell unit, and/ or a UPS and/ or a storage battery, both preferably rechargeable by the Fuel Cell itself.

8. A device (1) according to any previous claim, further comprising UV-C lamps (4).

9. A device according to any previous claim, further comprising a control unit (11) and programming means (12, 13) therefor.

10. A device (1) according to claim 7, wherein said programming means (12, 13) are selected from a display (12) and a computer (13) connectable with said control unit (11), or a combination thereof.

11. A process for sanitizing objects in a hospital, clinic, laboratory or other healthcare environment, in which a device (1) according to one of the preceding claims is used, wherein said device has a decontamination chamber (2); a source (8) of biocidal fluid; means (5) for feeding said fluid to said chamber (2) and at least one cylinder (7) of compressed medical air, the process comprising a step of decontaminating the objects in which a biocidal fluid is delivered into said decontamination chamber (2), **characterized in that** the decontamination chamber (2) is purged at the end of the decontaminating step, wherein said purging step comprises the steps of expelling air and biocidal fluid through at least one filter (9) and of delivering medical air into the chamber (2).

12. A process according to claim 11, wherein said decontamination is carried out at ambient pressure and said biocidal fluid is diffused into said chamber by recirculating air by means of a fan (15, 15') or similar recirculating means.

13. A process according to claim 11 or 12, wherein at least one of said recirculating means is used to expel air and biocidal fluid from said chamber (2).

14. A process according to claim 13, wherein said air and biocidal fluid are expelled from said chamber (2) through at least one filter (9) and wherein the passage through said filter (9) is prevented during said recirculating step by means of a shutter member (16) which is closed during the recirculating step and opened during the purging step.

15. A process according to any claim 11 to 14, wherein the same device (1) is used in different healthcare environments and a step is provided in which the device (1) is transferred from one to another of said environments.

16. A process according to claim 11, wherein said step of delivering a biocidal fluid into said decontamination chamber (2) and the step of delivering medical air into the chamber (2) occur at ambient pressure.

## Patentansprüche

1. Eine Vorrichtung (1) zum Desinfizieren von Gegenständen, umfassend eine Dekontaminationskammer (2); eine Quelle (8) eines bioziden Fluids; Mittel (5) zum Zuführen des Fluids zu der Kammer (2) und mindestens einer Druckluftquelle (7), **gekennzeichnet dadurch, dass** die Druckluft medizinische Luft ist, wobei die Quelle vorzugsweise ein Zylinder (7) medizinischer Druckluft ist.

2. Eine Vorrichtung (1) gemäß Anspruch 1, wobei das biozide Fluid eine Flüssigkeit ist und die Mittel (5) zum Zuführen zu der Dekontaminationskammer (2) mindestens einen Zerstäuber umfasst.

3. Eine Vorrichtung gemäß einem vorhergehenden Anspruch, ferner umfassend Mittel (15, 15') zum Umwälzen von Luft innerhalb der Kammer, um das vernebelte oder gasförmige Biozid innerhalb der Kammer zu diffundieren.

4. Eine Vorrichtung (1) gemäß einem vorhergehenden Anspruch, ferner umfassend Mittel zum Ausstoßen von Luft aus der Dekontaminationskammer (2), wobei die Ausstoßeinrichtung Aktivkohle- und/oder HEPA-Filter (9) umfasst.

5. Eine Vorrichtung gemäß den Ansprüchen 3 und 4, wobei die Umwälzmittel mindestens ein Gebläse (15, 15') umfassen und wobei das Gebläse oder eines der Gebläse (15') entweder neben oder in der Nähe eines Aktivkohle- oder HEPA-Filters angeordnet ist, und eines der Mittel (9) zum Ausstoßen von Luft und Biozid aus der Kammer (2) bildet.

6. Eine Vorrichtung gemäß Anspruch 5, wobei ein Verschlusselement (16) zum Abfangen eines Luftstroms zwischen dem Gebläse (15') und dem Filter (9) angeordnet ist, wobei das Verschlusselement beweglich ist, um den Luftdurchgang durch den Filter (9) nach außen zu öffnen oder zu schließen.

7. Eine Vorrichtung (1) gemäß einem vorhergehenden Anspruch, ferner umfassend eine vom Stromnetz unabhängige Stromquelle (10), vorzugsweise eine Wasserstoff-Brennstoffzelleneinheit und/oder eine USV und/oder eine Speicherbatterie, die beide vorzugsweise durch die Brennstoffzelle selbst wiederaufladbar sind.

8. Eine Vorrichtung (1) gemäß einem vorhergehenden Anspruch, ferner umfassend UV-C-Lampen (4).

9. Eine Vorrichtung gemäß einem vorhergehenden Anspruch, ferner umfassend eine Steuereinheit (11) und Programmiermittel (12, 13) dafür.

10. Eine Vorrichtung (1) gemäß Anspruch 7, wobei die Programmiermittel (12, 13) aus einer Anzeige (12) und einem Computer (13) ausgewählt sind, die mit der Steuereinheit (11) oder einer Kombination davon verbindbar sind.

11. Ein Verfahren zum Desinfizieren von Gegenständen in einem Krankenhaus, einer Klinik, einem Labor oder einer anderen Umgebung des Gesundheitswesens, in dem eine Vorrichtung (1) gemäß einem der vorhergehenden Ansprüche verwendet wird, wobei die Vorrichtung eine Dekontaminationskammer (2) aufweist; eine Quelle (8) eines bioziden Fluids; Mittel (5) zum Zuführen des Fluids zu der Kammer (2) und mindestens einen Zylinder (7) mit medizinischer Druckluft, wobei das Verfahren einen Schritt zum Dekontaminieren der Objekte umfasst, bei dem ein biozides Fluid in die Dekontaminationskammer (2) abgegeben wird, **dadurch gekennzeichnet, dass** die Dekontaminationskammer (2) am Ende des Dekontaminierungsschritts gespült wird, wobei der Spülschritt das Austreiben von Luft und biozidem Fluid durch mindestens einen Filter (9) und die Zuführung von medizinischer Luft in die Kammer (2) umfasst.

12. Ein Verfahren gemäß Anspruch 11, wobei die Dekontamination bei Umgebungsdruck durchgeführt wird und das biozide Fluid durch Umluft mit Hilfe eines Gebläses (15, 15') oder eines ähnlichen Umwälzmittels in die Kammer diffundiert wird.

13. Ein Verfahren gemäß Anspruch 11 oder 12, wobei mindestens eines der Umwälzmittel verwendet wird, um Luft und biozides Fluid aus der Kammer (2) auszutreiben.

14. Ein Verfahren gemäß Anspruch 13, wobei die Luft und das biozide Fluid durch mindestens einen Filter (9) aus der Kammer (2) ausgestoßen werden und wobei der Durchgang durch den Filter (9) während des Umwälzungsschritts mittels eines Verschlusselementes (16) verhindert wird, das während des Umwälzschritts geschlossen und während des Spülschritts geöffnet wird.

15. Ein Verfahren gemäß einem der Ansprüche 11 bis 14, wobei dieselbe Vorrichtung (1) in verschiedenen Umgebungen des Gesundheitswesens verwendet wird und ein Schritt bereitgestellt wird, in dem die Vorrichtung (1) von einer zu einer anderen dieser Umgebungen übertragen wird.

16. Ein Verfahren gemäß Anspruch 11, wobei der Schritt der Zuführung eines bioziden Fluids in die Dekontaminationskammer (2) und der Schritt der Zuführung medizinischer Luft in die Kammer (2) bei Umgebungstemperatur erfolgen.

## Revendications

1. Dispositif (1) pour désinfecter des objets, comprenant une chambre de décontamination (2) ; une source (8) de fluide biocide ; des moyens (5) pour alimenter en ledit fluide ladite chambre (2) et au moins une source (7) d'air comprimé, **caractérisé en ce que** ledit air comprimé est de l'air médical, de préférence ladite source étant une bouteille (7) d'air comprimé médical.

2. Dispositif (1) selon la revendication 1, dans lequel le fluide biocide est un liquide et lesdits moyens (5) pour l'alimenter dans ladite chambre de décontamination (2) comprennent au moins un nébuliseur.

3. Dispositif selon une quelconque revendication précédente, comprenant en outre des moyens (15, 15') pour remettre en circulation l'air à l'intérieur de ladite chambre afin de diffuser ledit biocide nébulisé ou gazeux à l'intérieur de ladite chambre.

4. Dispositif (1) selon une quelconque revendication précédente, comprenant en outre des moyens pour expulser l'air de la chambre de décontamination (2), lesdits moyens d'expulsion comprenant des filtres à charbon actif et/ou HEPA (9).

5. Dispositif selon les revendications 3 et 4, dans lequel lesdits moyens de remise en circulation comprennent au moins un ventilateur (15, 15'), et dans lequel ledit ventilateur ou l'un desdits ventilateurs (15') est positionné soit adjacent à, soit à proximité d'un filtre à charbon actif ou d'un filtre HEPA et il forme l'un desdits moyens (9) pour expulser l'air et le biocide de ladite chambre (2).

6. Dispositif selon la revendication 5, dans lequel un organe obturateur (16) pour intercepter un écoulement d'air est agencé entre ledit ventilateur (15') et ledit filtre (9), ledit organe obturateur étant mobile afin d'ouvrir ou fermer le passage d'air à travers ledit filtre (9) vers l'extérieur.

7. Dispositif (1) selon une quelconque revendication précédente, comprenant en outre une source d'alimentation (10) qui est indépendante du réseau électrique, de préférence une unité pile à hydrogène, et/ou une ASI et/ou une batterie secondaire, toutes deux étant de préférence rechargeables par la pile à hydrogène elle-même.

8. Dispositif (1) selon une quelconque revendication précédente, comprenant en outre des lampes UV-C (4).

9. Dispositif selon une quelconque revendication précédente, comprenant en outre une unité de commande (11) et des moyens de programmation (12, 13) pour celle-ci.

10. Dispositif (1) selon la revendication 7, dans lequel lesdits moyens de programmation (12, 13) sont sélectionnés parmi un dispositif d'affichage (12) et un ordinateur (13) pouvant être connecté à ladite unité de commande (11), ou une combinaison de ceux-ci.

11. Procédé pour désinfecter des objets dans un hôpital, une clinique, un laboratoire ou un autre environnement de santé, dans lequel un dispositif (1) selon l'une des revendications précédentes est utilisé, dans lequel ledit dispositif a une chambre de décontamination (2) ; une source (8) de fluide biocide ; des moyens (5) pour alimenter en ledit fluide ladite chambre (2) et au moins une bouteille (7) d'air médical comprimé, le procédé comprenant une étape de décontamination des objets dans laquelle un fluide biocide est distribué dans ladite chambre de décontamination (2), **caractérisé en ce que** la chambre de décontamination (2) est purgée à la fin de l'étape de décontamination, dans lequel ladite étape de purge comprend les étapes d'expulsion d'air et de fluide biocide à travers au moins un filtre (9) et de distribution d'air médical dans la chambre (2).

12. Procédé selon la revendication 11, dans lequel ladite décontamination est mise en œuvre à pression ambiante et ledit fluide biocide est diffusé dans ladite chambre par remise en circulation de l'air au moyen d'un ventilateur (15, 15') ou de moyens de remise en circulation similaires.

13. Procédé selon la revendication 11 ou 12, dans lequel au moins l'un desdits moyens de remise en circulation est utilisé pour expulser l'air et le fluide biocide de ladite chambre (2).

14. Procédé selon la revendication 13, dans lequel lesdits air et fluide biocide sont expulsés de ladite chambre (2) à travers au moins un filtre (9) et dans lequel le passage à travers ledit filtre (9) est empêché pendant ladite étape de remise en circulation au moyen d'un organe obturateur (16) qui est fermé pendant l'étape de remise en circulation et ouvert pendant l'étape de purge.

15. Procédé selon une quelconque revendication 11 à 14, dans lequel le même dispositif (1) est utilisé dans différents environnements de santé et une étape est fournie dans laquelle le dispositif (1) est transféré de l'un à l'autre desdits environnements.

16. Procédé selon la revendication 11, dans lequel ladite étape de distribution d'un fluide biocide dans ladite chambre de décontamination (2) et l'étape de distribution d'air médical dans la chambre (2) se produisent à pression ambiante.
